# FASCICULE DE BREVET EUROPEEN

(11) **EP 0 636 358 B1**
(45) Date de publication et mention de la délivrance du brevet: **29.12.1997**
(21) Numéro de dépôt: 94401646.8
(22) Date de dépôt: 19.07.1994
(51) Int. Cl.: A61K 7/09, A61K 7/13, A61K 7/135, A61K 7/06

(54) **Nouvelles compositions à base d'eau oxygénée et leur utilisation comme fixateurs pour permanentes**
Neue Zusammensetzungen auf der Basis von Wasserstoffperoxid und deren Verwendung als Fixiermittel für dauerhafte Haarverformungen
Novel compositions based on hydrogen peroxide and their use as fixers for permanent waving hair compositions

(30) Priorité: 28.07.1993 FR 9309287
(43) Date de publication de la demande: 01.02.1995
(73) Titulaire: L'OREAL, 75008 Paris (FR)
(72) Inventeur: Samain, Henri, F-91570 Bievres (FR)
(74) Mandataire: Andral, Christophe André Louis

(56) Documents cités:
- EP-A- 0 394 930
- EP-A- 0 512 879
- DE-U- 9 103 513
- CHEMICAL ABSTRACTS, vol. 112, no. 6, 5 Février 1990, Columbus, Ohio, US; abstract no. 42230m, A. MIZUNOYA 'cleansing agents for hair dyes' & JP-A-1 163 298 (...)
- PATENT ABSTRACTS OF JAPAN vol. 131, no. 82 (C-591) 27 Avril 1989 & JP-A-01 009 919 (NIKKA CHEM. IND. CO. LTD) 13 Janvier 1989

## Description

La présente invention concerne de nouvelles compositions dites oxydantes à base d'eau oxygénée, ainsi que l'utilisation de ces dernières dans des procédés de mise en forme et/ou de déformation permanente des matières kératiniques, en particulier des cheveux.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur (étape de réduction) puis, après avoir de préférence rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en plis.

Les compositions réductrices utilisables pour la mise en oeuvre de la première étape d'une opération de permanente contiennent généralement, à titre d'agents réducteurs, des sulfites, des bisulfites ou, de préférence, des thiols. Parmi ces derniers, ceux couramment utilisés sont la cystéïne et ses divers dérivés, la cystéamine et ses dérivés, l'acide thiolactique, l'acide thioglycolique ainsi que ses esters, notamment le monothioglycolate de glycérol, et le thioglycérol.

Pour réaliser l'étape de fixation susmentionnée, on fait le plus souvent appel, dans la pratique, à des compositions cosmétiquement acceptables à base d'eau oxygénée, qui conviennent en effet particulièrement bien.

Il est connu de CHEMICAL ABSTRACTS, vol. 112, n°6, 5 février 1990, Columbus, Ohio, US, abstract n°42230m, des compositions nettoyantes à base d'eau oxygénée, d'acide acétique, d'ammoniaque et d'alcool. Il est également connu de JP-A-01 009 919 un 〈〈 agent 〉〉 acide pour la déformation permanente des cheveux comprenant deux compositions distinctes, la première réductrice, la seconde oxydante et pouvant contenir du bromate de sodium ou de l'eau oxygénée. Il est connu de EP-A-0 394 930 une composition comprenant de l'eau oxygénée, de l'acide acétique et de l'ammoniaque utilisée dans un procédé de décoloration des cheveux.

Le problème des techniques de permanentes connues à ce jour est que leur application répétée sur les cheveux induit à la longue une modification importante du comportement de ces derniers, en particulier au niveau de leur aptitude à être par la suite correctement colorés. Ainsi, on observe tout d'abord que, sur des cheveux qui ont subi quelques opérations de permanente (de l'ordre de trois au maximum), la coloration sera beaucoup plus prononcée que celle obtenue sur les mêmes cheveux mais non permanentés; ceci pose donc un problème dans tous les cas où l'opération de coloration est conduite sur une chevelure à l'origine permanentée mais qui, entre temps, a aussi repoussée (mauvaise unisson entre les cheveux d'origine permanentés et les cheveux de repousse non permanentés). On observe par ailleurs que la coloration devient très difficile, voire impossible, si la chevelure à colorer a subi auparavant de nombreuses opérations de permanentes, en particulier plus de cinq opérations.

Un autre problème réside également dans le fait que, pour diverses raisons, il est généralement nécessaire de tamponner le pH de la composition réductrice par ajout de certains additifs, et en particulier de produits carbonatés comme par exemple l'acide ou le gaz carbonique, les carbonates ou bicarbonates d'alcalins ou d'ammonium, les carbonates organiques tels que notamment le carbonate de guanidine.

Or, il s'avère malheureusement que l'application répétée des opérations de mises en forme/déformation permanentes au moyen de compositions réductrices carbonatées associées à des compositions oxydantes à base d'eau oxygénée, entraine à la longue une altération progressive et marquée de la qualité du cheveu, en particulier au niveau de la douceur des fibres qui ont tendance à devenir de plus en plus rêches.

La présente invention a notamment pour but de résoudre les problème ci-dessus.

Ainsi, à la suite d'importantes recherches menées sur la question, il a été trouvé par la Demanderesse que l'utilisation de certaines compositions oxydantes particulières et nouvelles en soi à base d'eau oxygénée pouvait permettre de remédier avec succès aux divers inconvénients liés de manière inhérente à l'application répétée sur les cheveux de compositions réductrices, en particulier carbonatées, et de compositions oxydantes oxygénées.

Cette découverte est à la base de la présente invention.

Ainsi, au titre de l'un des objets de la présente invention, il est maintenant proposé de nouvelles compositions (ci-après dénommées compositions "oxydantes" pour les besoins de l'exposé) à base d'eau oxygénée, lesdites compositions étant caractérisées par le fait qu'elles comprennent en outre (i) au moins un acide carboxylique et/ou l'un de ses sels associés, et (ii) au moins un composé aminé basifiant.

L'invention a également pour objet l'utilisation desdites compositions comme, ou pour la fabrication de, fixateurs pour permanentes.

L'invention a également pour objet un dispositif à plusieurs compartiments ou "kit", caractérisé par le fait qu'il comprend dans un premier compartiment une composition réductrice, en particulier carbonatée, et dans un deuxième compartiment une composition oxydante conforme à l'invention, ledit dispositif étant réalisé en vue notamment de mettre en oeuvre un procédé de traitement pour la déformation et/ou la mise en forme permanente des matières kératiniques, en particulier des cheveux.

L'invention a également pour objet un procédé de traitement pour la déformation et/ou la mise en forme permanente des matières kératiniques, en particulier des cheveux, du type consistant dans une première étape à réduire les liaisons disulfures de la kératine par application d'une composition réductrice, en particulier carbonatée, puis, dans une seconde étape, à reformer lesdites liaisons par application d'une composition oxydante à base d'eau oxygénée, caractérisé par le fait que ladite composition oxydante comprend (i) au moins un acide carboxylique et/ou l'un de ses sels associés, et (ii) au moins un composé aminé basifiant.

La présente invention a encore pour objet un tel procédé dans lequel on utilise un 〈〈 kit 〉〉 tel que défini ci-dessus.

D'autres caractéristiques, aspects, objets et avantages de l'invention apparaitront encore plus clairement à la lecture de la description détaillée qui va suivre, ainsi que des divers exemples concrets, mais nullement limitatifs, destinés à l'illustrer.

Bien que l'exposé qui suit s'articule essentiellement autour du cas particulier du traitement du cheveu, on notera ici que la présente invention n'y est nullement limitée et qu'elle est au contraire applicable à toute matière kératinique en général, notamment cils, moustaches, poils, laine et autres.

A titre d'acides carboxyliques utilisables dans les compositions selon l'invention, on peut plus particulièrement citer les acides lactique, tartrique, acétique, glycolique et citrique. Par acides carboxyliques, on entend donc ici désigner et couvrir notamment les acides carboxyliques simples, les acides polycarboxyliques et les acides (poly)hydroxy(poly)carboxyliques, qui peuvent bien entendu être pris seuls ou en mélanges.

D'une manière générale, tout composé présentant au moins une fonction carboxylique 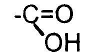 et qui est capable d'abaisser (dans sa zone acide) le pH d'une solution d'eau oxygénée, est susceptible de convenir dans le cadre de la présente invention. De préférence, on retiendra les acides qui sont cosmétiquement compatibles et acceptables avec les cheveux, la peau et/ou le cuir chevelu.
Selon un mode particulièrement préféré de réalisation de la présente invention, l'acide mis en oeuvre est l'acide citrique.

Comme indiqué précédemment, il convient de noter que les acides carboxyliques utilisés dans l'invention peuvent être présents dans les compositions finales, et ceci partiellement ou totalement, sous la forme d'un de leurs sels associés, cette présence et son ampleur dépendant en particulier du pH final imposé à ladite composition.

A titre de composés aminés basifiants convenant aux compositions oxydantes selon l'invention, on peut citer, entre autres, l'ammoniaque, les (poly)amines primaires, secondaires ou tertiaires, telles que par exemple a monoéthanolamine, la diéthanolamine, la triéthanolamine, l'isopropanolamine et la propanediamine-1,3. D'une manière générale, on peut aussi utiliser toute diamine répondant à la formule suivante : 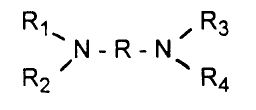 dans laquelle R est un reste propylène éventuellement substitué par un groupement hydroxyle ou un radical alkyle en C₁-C₄; et R₁, R₂, R₃ et R₄ représentent, simultanément ou indépendamment l'un de l'autre, l'hydrogène, un radical alkyle ou hydroxyalkyle en C₁-C₄.

De préférence, on met en oeuvre l'ammoniaque ou la monoéthanolamine.

Tous les composé aminés basifiants ci-dessus mentionnés peuvent bien entendu être utilisés seuls ou en mélanges.

On entend ici désigner par basifiant le fait que le composé aminé est capable d'élever le pH naturel de l'eau (pH 7) à une valeur supérieure à 7.

Selon l'invention, les compositions oxydantes peuvent être obtenues soit en procédant d'abord à l'ajout de l'acide carboxylique (qui peut alors se présenter sous la forme d'un liquide ou d'un solide) dans une solution d'eau oxygénée, puis à l'ajout dans le mélange résultant du composé aminé (solide ou liquide), ou inversement. L'introduction simultanée de l'acide et du composé aminé dans la solution d'eau oxygénée est également possible.

Dans le cas particulier où les compositions selon l'invention sont destinées à être utilisées comme agents de fixation pour permanentes, lesdites compositions présentent en outre, de préférence, au moins l'une des caractéristiques complémentaires suivantes:
- la concentration en acide(s) carboxylique(s) et/ou en leurs sels associés y est comprise entre 0,1N et 2N, et encore plus préférentiellement entre 0,2N et 1N
- la concentration en composé(s) aminé(s) y est ajustée de manière telle que le pH final de la composition soit compris entre 2,8 et 6, et encore plus préférentiellement entre 4 et 5,5
- la concentration en eau oxygénée varie de 1 à 20 volumes, et est encore plus préférentiellement comprise entre 1 et 10 volumes
- la composition contient des adjuvents classiques et usuels destinés à la rendre cosmétiquement acceptable, lesdits adjuvents étant par exemple choisis choisis, seuls ou en mélange, parmi des agents tensio-actifs de type non-ionique, anionique, cationique ou amphotère, des agents traitants, des ingrédients actifs, des agents antichutes, des agents anti-pelliculaires, des épaississants, des agents de suspension, des agents sequestrants, des agents opacifiants, des colorants, des filtres solaires, des parfums et des conservateurs, certains de ces additifs allant d'ailleurs être un peu plus détaillés dans l'exposé qui va suivre relatif aux compositions réductrices, en particulier carbonatées, susceptibles d'être utilisées dans le cadre du procédé selon l'invention et/ou du "kit" selon l'invention, et dans lesquelles, classiquement, on peut retrouver ces mêmes additifs.
- la composition se présente sous la forme d'une lotion, épaissie ou non, d'un lait, d'une crème, d'un gel, ou de toute autre forme appropriée.

De manière la plus générale, les compositions réductrices utilisables soit dans la réalisation d'un kit conforme à l'invention soit pour la mise en oeuvre de la première étape de l'opération de permanente d'un procédé conforme à l'invention, peuvent consister en toute composition déja connue en soi comme composition réductrice. En particulier, elles peuvent contenir, à titre d'agents réducteurs, des sulfites et/ou des bisulfites (notamment d'alcalins, d'alcalino-terreux ou d'ammonium) ou, de préférence, des thiols. Parmi ces derniers, ceux les plus couramment utilisés sont la cystéïne et ses divers dérivés (notamment la N-acétylcystéïne), la cystéamine et ses divers dérivés (notamment ses dérivés acylés en C₁-C₄ tels que la N-acétyl cystéamine ou la N-propionyl cystéamine), l'acide thiolactique et ses esters (notamment le monothiolactate de glycérol), l'acide thioglycolique ainsi que ses esters, notamment le monothioglycolate de glycérol ou de glycol, et le thioglycérol. On peut également mentionner les réducteurs suivants : les N-mercaptoalkylamides de sucres tels que le N-(mercapto-2-éthyl)gluconamide, l'acide β- mercaptopropionique et ses dérivés, l'acide thiomalique, la panthétéïne, les N-(mercaptoalkyl)ω-hydroxyalkylamides décrits dans la demande de brevet EP-A- 354 835 et les N-mono- ou N,N-dialkylmercapto 4-butyramides décrits dans la demande de brevet EP-A- 368 763, les aminomercaptoalkylamides décrits dans la demande de brevet EP-A-432 000 et les alkylaminomercaptoalkylamides décrits dans la demande de brevet EP-A- 514 282, le mélange de thioglycolate d'hydroxy-2 propyle (2/3) et de thioglycolate d'hydroxy-2 méthyl-1 éthyle (67/33) décrit dans la demande de brevet FR-A- 2 679 448..

Comme déja souligné ci-avant, ces agents réducteurs sont généralement mis en oeuvre dans des compositions cosmétiquement acceptables, lesquelles sont par ailleurs déjà bien connues en soi dans l'état de l'art existant des formulations frisantes destinées à réaliser la première étape (réduction) d'une opération de permanente. Ainsi, à titre d'additifs usuels et classiques, utilisables seuls ou en mélanges, on peut plus particulièrement mentionner les agents tensioactifs de type non-ionique, anionique, cationique ou amphotère et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque la composition réductrice contient au moins un agent tensioactif, celui-ci est généralement présent à une concentration maximale de 30 % en poids, et de préférence comprise entre 0,5 et 10% en poids, par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non-ionique ou amphotère.

Parmi les agents traitants particulièrement préférés, on peut notamment citer ceux décrits dans les demandes de brevets français n° 2 598 613 et 2 470 596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2 535 730, les polyorganosiloxanes à groupements aminoalkyles modifiés par des groupements alcoxycarbonyalkyles tels que ceux décrits dans le brevet US n° 4 749 732, des polyorganosiloxanes tels que le copolymère polydiméthylsiloxane-polyoxyalkyle du type Diméthicone Copolyol, un polyd iméthylsiloxane à groupements terminaux stéaroxy- (stéaroxydiméthicone), un copolymère polydiméthylsiloxane-dialkylammonium acétate ou un copolymère polydiméthyl-siloxane polyalkylbétaïne décrits dans la demande de brevet britannique n° 2 197 352, des polysiloxanes organo modifés par des groupements mercapto ou mercaptoalkyles tels que ceux décrits dans le brevet français n° 1 530 369 et dans la demande de brevet européen n° 295 780, ainsi que des silanes tels que le stéaroxytriméthylsilane.

La composition réductrice peut également contenir d'autres ingrédients traitants tels que des polymères cationiques tels que ceux utilisés dans les compositions des brevets français n° 79.32078 (FR-A- 2 472 382) et 80.26421 (FR-A- 2 495 931), ou encore des polymères cationiques du type ionène tels que ceux utilisés dans les compositions du brevet luxembourgeois n° 83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO2/PDMS (polydiméthylsiloxane), le diméthylisosorbitol, I'urée et ses dérivés, la pyrrolidone, les N-alkyl-pyrrolidones, la thiamorpholinone, les alkyléthers d'alkyléneglycol ou de dialkylèneglycol tels que par exemple le monométhyléther de propylène glycol, le monométhyléther de dipropyléne glycol, le monoéthyléther de l'éthylèneglycol et le monoéthyléther du diéthylèneglycol, des alcanediols en C₃-C₆ tels que par exemple le propanediol-1,2 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

Dans des compositions réductrices de permanente, les agents réducteurs tels que ceux mentionnés ci-avant sont généralement présents à une concentration qui peut être comprise entre 1 et 30 % en poids, et de préférence entre 5 et 20 % en poids par rapport au poids total de la composition réductrice.

La composition réductrice peut se présenter sous la forme d'une lotion, épaissie ou non, d'une crème, d'un gel, ou de toute autre forme appropriée.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol, ou de l'isopropanol ou encore du glycérol à une concentration maximale de 20 % par rapport au poids total de la composition.

Le véhicule des compositions est de préférence l'eau ou une solution hydroalcoolique d'un alcool inférieur tel que l'éthanol, l'isopropanol ou le butanol.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lisse pendant le temps de pose.

Enfin, les compositions réductrices peuvent également contenir au moins un disulfure connu pour son utilisation dans une composition réductrice pour permanente auto-neutralisante.

Parmi de tels disulfures connus, on peut notamment mentionner l'acide dithioglycolique, le dithioglycérol, la cystamine, la N, N'-diacétyl-cystamine, la cystine, la pantéthine, et les disulfures des N-(mercapto-alkyl)ω-hydroxyalkylamides décrits dans la demande de brevet européen EP 354 835, les disulfures des N-mono ou N,N-dialkylmercapto-4 butyramides décrits dans la demande de brevet EP 368 763, les disulfures des aminomercapto-alkylamides décrits dans la demande de brevet EP 432 000, et les disulfures des alkylaminomercaptoalkylamides décrits dans la demande de brevet EP 514 282. Ces disulfures sont généralement présents dans un rapport molaire de 0,5 à 2,5, et de préférence de 1 à 2, par rapport à l'agent réducteur (voir brevet US 3 768 490).

Les pH des compositions réductrices peuvent être ajustés classiquement par ajout soit d'agents basifiants, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine (compositions réductrices carbonatées) ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique ou l'acide borique.

Un procédé particulier de mise en oeuvre des compositions réductrices et oxydantes décrites précédemment va maintenant être donné à titre d'application. Ce procédé correspond à un procédé de traitement pour la déformation et/ou la mise en forme, et ceci de manière permanente, des matières kératiniques, en particulier des cheveux, et il comprend alors les étapes essentielles suivantes :
(i) on applique sur la matière kératinique à traiter une composition réductrice notamment telle que ci-dessus définie, et en particulier carbonatée, les moyens (tels que par exemple rouleaux, bigoudis et analogues) nécessaires à la mise sous tension mécanique de la matière kératinique étant mis en oeuvre avant, pendant ou après ladite application,
(ii) puis on rince la matière kératinique ainsi traitée,
(iii) on applique sur la matière kératinique ainsi rincée une composition oxydante conforme à l'invention,
(iv) puis on sépare la matière kératinique ainsi traitée des moyens (rouleaux et autres) de mise sous tension utilisés à l'étape (i),
(v) et enfin on rince à nouveau la matière kératinique.
Conformément à la première étape du procédé (étape (i)), les compositions contenant le ou les agents réducteurs tels que mentionnés ci-avant sont appliquées sur les cheveux à traiter, lesquels auront été de préférence préalablement mouillés.
Cette application peut être réalisée avant, pendant ou après l'habituelle étape de mise sous tension des cheveux sous une forme correspondant à la forme finale désirée pour ces derniers (boucles par exemple), cette étape pouvant elle-même être mise en oeuvre par tout moyen, mécanique notamment, approprié et connu en soi pour maintenir sous tension des cheveux, tels que par exemple rouleaux, bigoudis et analogues (diamètre des rouleaux: généralement de 4 à 20 mm).

Avant de procéder à l'étape suivante de rinçage, il convient, de manière classique, de laisser reposer pendant quelques minutes, généralement entre 2 et 30 mn, de préférence entre 5 et 20 mn, la chevelure sur laquelle a été appliquée la composition réductrice, et ceci de façon à bien laisser le temps au réducteur d'agir correctement sur les cheveux. Cette phase d'attente est généralement conduite en laissant reposer à l'air libre (température ambiante) la chevelure traitée. Une phase d'attente conduite à température plus élevée n'est pas exclue, et convient également (casque de coiffure, dispensateur de rayons infra-rouges, ou tout autre appareil chauffant). Pendant cette phase d'attente, on prend soin que les cheveux ne sèchent pas complètement et restent ainsi humides jusqu'au moment de la mise en oeuvre de l'étape suivante (à cet effet, utilisation possible de bonnets, de gels de protection par exemple).

Dans la deuxième étape du procédé (étape (ii)), les cheveux imprégnés de composition réductrice sont donc ensuite rincés soigneusement, généralement à l'eau.

Puis, dans une troisième étape (étape (iii)), on applique sur les cheveux ainsi rincés une composition oxydante conforme à l'invention dans le but de fixer la nouvelle forme imposée aux cheveux. Comme dans le cas de l'application de la composition réductrice, la chevelure sur laquelle a été appliquée la composition oxydante est ensuite, et de manière classique, laissée dans une phase de repos ou d'attente qui dure quelques minutes, généralement entre 3 et 20 mn, de préférence entre 5 et 15 mn.

Dans la quatrième étape du procédé (étape (iv)), on enlève de la chevelure les moyens mécaniques (rouleaux, bigoudis et analogues) qui maintenaient sous tension et dans la forme désirée les cheveux tout au long du traitement.

Enfin, dans la dernière étape du procédé (étape (v)), on rince soigneusement et abondamment à l'eau la chevelure, ce par quoi l'on peut obtenir finalement, après séchage (naturel à l'air libre ou au moyen d'un appareil chauffant) une chevelure présentant par exemple de belles boucles permanentes (en particulier résistantes à l'eau).

Même répété plusieurs fois sur la même chevelure, et même avec des compositions réductrices de type carbonatées, le procédé conduit toujours à des cheveux présentant d'une part une bonne douceur, proche de la douceur d'origine et, d'autre part, un comportement à la coloration très correct.

Des exemples concrets illustrant l'invention vont maintenant être donnés. Aux fins d'une comparaison significative, pour un exemple donné, les mêmes cheveux de départ (avant traitement) ont été utilisés pour conduire l'essai conforme à l'invention et l'essai comparatif. Par contre, les cheveux de départ étaient différents d'exemple à exemple (exemple 1 : cheveux fortement décolorés; exemple 2 : cheveux naturels châtains; exemple 3 : cheveux naturels châtains; exemple 4 : cheveux décolorés ; exemple 5 : cheveux gris européen naturels).

La composition réductrice carbonatée constamment utilisée pour conduire les exemples 1 à 4 présentait les caractéristiques suivantes :

| | |
|---|---|
| - acide thioglycolique | 9,1 g |
| - acide mercaptopropionique | 2,2 g |
| - carbonate d'ammonium | 7,3 g |
| - ammoniaque qsp | pH 8,5 |
| - parfum qs | |
| - eau déminéralisée qsp | 100 g |

La composition réductrice non carbonatée utilisée à l'exemple 5 présentait, quant à elle, les caractéristiques suivantes :

| | |
|---|---|
| - acide thioglycolique | 9,2 g |
| - ammoniaque qsp | pH 8,5 |
| - eau déminéralisée qsp | 100 g |

En outre, pour tous les exemples, le mode opératoire commun utilisé pour conduire chaque permanente était le suivant : on applique sur des cheveux enroulés et humides (diamètre des rouleaux : 9 mm) la composition réductrice, puis on pose un bonnet en plastique sur la chevelure et on attend 15 mn; puis on enlève le bonnet et on rince abondamment à l'eau; puis on applique sur les cheveux la composition oxydante et on attend 10 mn; on retire ensuite les rouleaux, puis on rince une nouvelle fois à l'eau et enfin on sèche les cheveux.

### Exemple 1

On a réalisé, en suivant le mode opératoire donné ci-avant, quatre permanentes successives (shampooing + séchage au casque entre chaque permanente), en utilisant à chaque fois comme composition fixante une composition oxydante conforme à l'invention qui avait les caractéristiques suivantes :

| | |
|---|---|
| - eau oxygénée qsp | 8 V |
| - acide citrique | 4 g |
| - ammoniaque (20%) qsp | pH 4,5 |
| - eau déminéralisée qsp | 100 g |

On obtient ainsi finalement une mèche n°1.

A titre comparatif, on a reproduit le protocole opératoire ci-dessus (quatre opérations de permanente) mais en mettant cette fois en oeuvre la composition fixante suivante (non conforme à l'invention) :

| | |
|---|---|
| - eau oxygénée qsp | 8 V |
| - acide citrique qsp | pH 4,5 |
| - eau déminéralisée qsp | 100 g |

On obtient ainsi finalement une mèche n°2.

On constate que la mèche n°1 est beaucoup plus douce et moins fripée que la mèche n°2.

Ainsi, un panel de 10 personnes ont jugé ces deux mèches sur les critères de douceur, lissage et fripage (test d'évaluation sensoriel), en donnant des notes de 0 à 5. 0 correspond à un état inacceptable, alors que 5 correspond à un excellent résultat. Le lissage rend compte de la sensation tactile et le fripage correspond à la sensation visuelle.
Les moyennes ont été les suivantes :

| Critère | Mèche n°1 | Mèche n°2 |
|---|---|---|
| Douceur | 3,4 | 1,75 |
| Fripage | 3,15 | 1,5 |
| Lissage | 3,15 | 1,55 |

### Exemple 2

On a procédé comme à l'exemple 1, à ces différences près que (i) la composition oxydante de comparaison (non conforme à l'invention) qui a été mise en oeuvre avait cette fois les caractéristiques suivantes:

| | |
|---|---|
| - eau oxygénée qsp | 8 V |
| - acide citrique | 4 g |
| - eau déminéralisée qsp | 100 g |

et que (ii) l'opération de permanente n'a été répétée que deux fois.

On a ainsi obtenu deux nouvelles mèches (mèche n°3, conforme à l'invention; mèche n° 4, comparative) pour lesquelles les résultats du panel (identique à celui de l'exemple 1) ont été les suivants :

| Critère | Mèche n°3 | Mèche n°4 |
|---|---|---|
| Douceur | 3,6 | 2,9 |
| Fripage | 4,3 | 2,5 |
| Lissage | 3,85 | 2,85 |

### Exemple 3

On a procédé comme à l'exemple 1, à cette différence près que la composition oxydante de comparaison (non conforme à l'invention) qui a été ici mise en oeuvre avait les caractéristiques suivantes :

| | |
|---|---|
| - eau oxygénée qsp | 8 V |
| - acide citrique | 4g |
| - soude qsp | pH 4,5 |
| - eau déminéralisée qsp | 100 g |

On a ainsi obtenu deux nouvelles mèches (mèche n°5, conforme à l'invention; mèche n°6, comparative) notées par le panel de la manière suivante :

| Critère | Mèche n°5 | Mèche n°6 |
|---|---|---|
| Douceur | 3,75 | 3 |
| Fripage | 3,9 | 1,65 |
| Lissage | 3,65 | 2,45 |

### Exemple 4

En reproduisant le mode opératoire de l'exemple 1, les mêmes résultats de douceur, fripage et lissage ont été obtenus en mettant en oeuvre les compositions oxydantes conformes à l'invention suivantes :

| Composition A | |
|---|---|
| - eau oxygénée (60% de matière active) | 4 g |
| - acide citrique | 2 g |
| - ammoniaque qs | pH 4,5 |
| - eau déminéralisée qs | 100 g |

| Composition B | |
|---|---|
| - eau oxygénée (60% de matière active) | 4,8 g |
| - acide citrique | 1,8 g |
| - monoéthanolamine qs | pH 4,2 |
| - eau déminéralisée qs | 100 g |

| Composition C | |
|---|---|
| - eau oxygénée (60% de matière active) | 3,6 g |
| - acide citrique | 3,2 g |
| - triéthanolamine qs | pH 4,8 |
| - eau déminéralisée qs | 100 g |

| Composition D | |
|---|---|
| - eau oxygénée (60% de matière active) | 4 g |
| - acide citrique | 3 g |
| - propane 1,3 diamine qs | pH 5 |
| - eau déminéralisée qs | 100 g |

| Composition E | |
|---|---|
| - eau oxygénée (60% de matière active) | 4 g |
| - acide lactique | 1,6 g |
| - ammoniaque qs | pH 4,5 |
| - eau déminéralisée qs | 100 g |

| Composition F | |
|---|---|
| - eau oxygénée (60% de matière active) | 6 g |
| - acide lactique | 2 g |
| - monoéthanolamine qs | pH 4,2 |
| - eau déminéralisée qs | 100 g |

| Composition H | |
|---|---|
| - eau oxygénée (60% de matière active) | 4 g |
| - acide citrique | 1 g |
| - acide lactique | 1 g |
| - ammoniaque qs | pH 4,8 |
| - eau déminéralisée qs | 100 g |

| Composition I | |
|---|---|
| - eau oxygénée (60% de matière active) | 4,5 g |
| - acide glycolique | 1,5 g |
| - ammoniaque qs | pH 4,5 |
| - eau déminéralisée qs | 100 g |

| Composition J | |
|---|---|
| - eau oxygénée (60% de matière active) | 2,8 g |
| - acide glycolique | 2 g |
| - monoéthanolamine qs | pH 4,5 |
| - eau déminéralisée qs | 100 g |

| Composition K | |
|---|---|
| - eau oxygénée (60% de matière active) | 4 g |
| - acide citrique | 1,8 g |
| - acide glycolique | 1 g |
| - ammoniaque qs | pH 4,5 |
| - eau déminéralisée qs | 100 g |

| Composition L | |
|---|---|
| - eau oxygénée (60% de matière active) | 4 g |
| - acide tartrique | 2,2 g |
| - ammoniaque qs | pH 4,5 |
| - eau déminéralisée qs | 100 g |

| Composition M | |
|---|---|
| - eau oxygénée (60% de matière active) | 4 g |
| - acide acétique | 1,6 g |
| - monoéthanolamine qs | pH 4,5 |
| - eau déminéralisée qs | 100 g |

### Exemple 5

Cet exemple a pour but de montrer l'intérêt des compositions selon l'invention dans le cadre de la mise en oeuvre de permanentes non carbonatées.

En suivant le même mode opératoire que celui donné ci-avant, on fait subir à une mêche de cheveux une répétition (en nombre variable) d'opérations de permanentes identiques, ces dernières étant conduites systématiquement avec la composition réductrice non carbonatée donnée en début d'exemples mais avec une composition oxydante qui peut être soit conforme à l'invention (Formulation n°1) soit non conforme à l'invention (Formulation n°2).

La mêche de cheveux finalement obtenue est ensuite traitée par une teinture commerciale (hauteur de ton blond, nuance rouge) en vue de leur coloration.

On note les résultats tinctoriaux après 30 mn d'application de la teinture, par mesure des coordonnées trichromatiques L, a, b, des cheveux (colorimètre MINOLTA CHROMA METER 2002).

Les résultats sont rassemblés dans le tableau donné ci-après.

Les formulations oxydantes mises en oeuvre avaient les caractéristiques suivantes :

| Formulation n°1 (invention) | |
|---|---|
| - eau oxygénée (60% de matière active) | 4 g |
| - acide citrique | 2 g |
| - ammoniaque qs | pH 4,5 |
| - eau déminéralisée qs | 100 g |

| Formulation n°2 (comparatif) | |
|---|---|
| - eau oxygénée (60% de matière active) | 4,8 g |
| - acide citrique qs | pH 3 |
| - eau déminéralisée qs | 100 g |

On s'aperçoit que l'utilisation d'un fixateur conforme à l'invention (ici, acide citrique + ammoniaque) permet de retarder le phénomène de sensibilisation des fibres (mèche n°3) amenant dans le cas classique à une impossibilité de teindre les cheveux. Le paramètre L nettement plus élevé dans le cas de la mèche n°6 que dans le cas de la mèche n°4 provient du fait que cette dernière mèche ne se colore plus normalement. Ce phénomène n'est pas observé lorsque les mèches sont traitées par des permanentes mettant en jeu des fixateurs conformes à l'invention, puisque les résultats tinctoriaux des mèches n°1, 2 et 3 sont très proches.

**Tableau**

| Mèche | nombre de permanente | Formulation oxydante | Coordonnées chromatiques | | |
|---|---|---|---|---|---|
| | | | L | a | b |
| 1 (inv) | 2 | 1 | 30,0 | 16,0 | 10,6 |
| 2 (inv) | 5 | 1 | 28,9 | 12,2 | 7,1 |
| 3 (inv) | 10 | 1 | 27,6 | 11,4 | 7,7 |
| 4 (comp) | 2 | 2 | 29,6 | 15,6 | 9,9 |
| 5 (comp) | 5 | 2 | 29,7 | 15,1 | 10,6 |
| 6 (comp) | 10 | 2 | 35,3 | 11,7 | 12,1 |

## Revendications

1. Procédé de traitement pour la déformation et/ou la mise en forme permanente des matières kératiniques, du type consistant dans une première étape à réduire les liaisons disulfures de la kératine par application d'une composition réductrice puis, dans une seconde étape, à reformer lesdites liaisons par application d'une composition oxydante à base d'eau oxygénée, caractérisé par le fait que ladite composition oxydante comprend (i) au moins un acide carboxylique et/ou l'un de ses sels associés, et (ii) au moins un composé aminé basifiant.

2. Procédé selon la revendication 1, caractérisé en ce que ledit acide carboxylique est choisi, seul ou en mélanges, parmi les acides carboxyliques simples,les acides polycarboxyliques,les acides (poly)hydroxy(poly)carboxyliques.

3. Procédé selon la revendication 2, caractérisé en ce que ledit acide carboxylique est choisi, seul ou en mélanges, parmi les acides lactique, tartrique, acétique, glycolique et citrique.

4. Procédé selon la revendication 3, caractérisé en ce que ledit acide carboxylique est l'acide citrique.

5. Procédé selon l'une quelconque des revendications précédentes, caractérisé en ce que ledit composé aminé basifiant est choisi, seul ou en mélange, parmi l'ammoniaque et les (poly)amines primaires, secondaires ou tertiaires.

6. Procédé selon la revendication 5, caractérisé en ce que lesdites polyamines répondent à la formule générale : 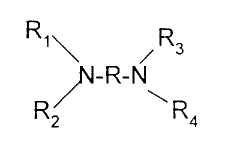 dans laquelle R est un reste propylène éventuellement substitué par un groupement hydroxyle ou un radical alkyle en C₁-C₄ ; et R₁, R₂, R₃ et R₄ représentent, simultanément ou indépendamment l'un de l'autre, l'hydrogène, un radical alkyle ou hydroxyalkyle en C₁-C₄.

7. Procédé selon l'une des revendications 5 ou 6, caractérisé en ce que lesdites (poly)amines sont choisies, seules ou en mélanges, parmi la monoéthanolamine, la diéthanolamine, la triéthanolamine, l'isopropanolamine et la propanediamine-1,3.

8. Procédé selon l'une quelconque des revendications précédentes, caractérisé en ce que la concentration en acide(s) carboxylique(s) et/ou en leurs sels associés au sein de la composition oxydante est comprise entre 0,1N et 2N.

9. Procédé selon la revendication 8, caractérisé en ce que ladite concentration est comprise entre 0,2N et 1N.

10. Procédé selon l'une quelconque des revendications précédentes, caractérisé en ce que le pH de la composition oxydante est compris entre 2,8 et 6.

11. Procédé selon la revendication 10, caractérisé en ce que ledit pH est compris entre 4 et 5,5.

12. Procédé selon l'une quelconque des revendications précédentes, caractérisé en ce que la concentration en eau oxygénée dans la composition oxydante est comprise entre 1 et 20 volumes.

13. Procédé selon la revendication 12, caractérisé en ce que ladite concentration est comprise entre 1 et 10 volumes.

14. Procédé selon l'une quelconque des revendications précédentes, caractérisé en ce la composition oxydante contient des adjuvants destinés à le rendre cosmétiquement acceptable.

15. Procédé selon la revendication 14, caractérisée en ce que lesdits adjuvants sont choisis, seuls ou en mélanges, parmi des agents tensio-actifs de type non-ionique, anionique, cationique ou amphotère, des agents traitants, des ingrédients actifs, des agents antichutes, des agents anti-pelliculaires, des épaississants, des agents de suspension, des agents sequestrants, des agents opacifiants, des colorants, des filtres solaires, des parfums et des conservateurs.

16. Procédé selon l'une quelconque des revendications précédentes, caractérisée en ce la composition oxydante se présente sous la forme d'une lotion, épaisse ou non, d'un lait, d'une crème ou d'un gel.

17. Procédé selon l'une quelconque des revendications précédentes, caractérisé en ce que ladite composition réductrice est une composition réductrice carbonatée.

18. Procédé selon l'une quelconque des revendications précédentes, caractérisé par le fait qu'il comprend les étapes suivantes :
(i) on applique sur la matière kératinique à traiter la composition réductrice, les moyens nécessaires à la mise sous tension mécanique de la matière kératinique étant mis en oeuvre avant, pendant ou après ladite application,
(ii) puis on rince la matière kératinique ainsi traitée,
(iii) on applique sur la matière kératinique ainsi rincée la composition oxydante,
(iv) puis on sépare la matière kératinique ainsi traitée des moyens de mise sous tension utilisés à l'étape (i)
(v) et enfin on rince à nouveau la matière kératinique.

19. Procédé selon l'une quelconque des revendications précédentes, caractérisé en ce que la matière kératinique à traiter consiste en des cheveux.

20. Dispositif à plusieurs compartiments ou "kit", caractérisé par le fait qu'il comprend dans un premier compartiment une composition réductrice, et, dans un deuxième compartiment, une composition oxydante telle que définie à l'une quelconque des revendications 1 à 16.

21. Dispositif selon la revendication 20, caractérisé en ce que ladite composition réductrice est une composition réductrice carbonatée apte et destinée à la mise en oeuvre de la première étape d'une opération de permanente.

22. Procédé de traitement pour la déformation et/ou la mise en forme permanente des matières kératiniques, du type consistant dans une première étape à réduire les liaisons disulfures de la kératine par application d'une composition réductrice puis, dans une seconde étape, à reformer lesdites liaisons par application d'une composition oxydante à base d'eau oxygénée, caractérisé par le fait que l'on utilise un "kit" tel que défini aux revendications 20 ou 21.

## Claims

1. Treatment process for the permanent-reshaping and/or -shape-stabilizing of keratinous materials, of the type consisting, in a first step, in reducing the disulphide bonds of keratin by application of a reducing composition, and then, in a second step, in re-forming the said bonds by application of a hydrogen peroxide-based oxidizing composition, characterized in that the said oxidizing composition comprises (i) at least one carboxylic acid and/or one of its associated salts, and (ii) at least one alkalinizing amino compound.

2. Process according to Claim 1, characterized in that the said carboxylic acid is chosen from simple carboxylic acids, polycarboxylic acids and (poly)hydroxy(poly)carboxylic acids, alone or mixed.

3. Process according to Claim 2, characterized in that the said carboxylic acid is chosen from lactic, tartaric, acetic, glycolic and citric acids, alone or mixed.

4. Process according to Claim 3, characterized in that the said carboxylic acid is citric acid.

5. Process according to any one of the preceding claims, characterized in that the said alkalinizing amino compound is chosen from aqueous ammonia and primary, secondary or tertiary (poly)amines, alone or mixed.

6. Process according to Claim 5, characterized in that the said polyamines correspond to the general formula: 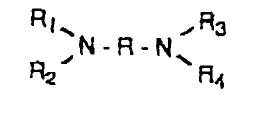 in which R is a propylene residue optionally substituted with a hydroxyl group or a C₁-C₄ alkyl radical; and R₁, R₂, R₃ and R₄ represent, simultaneously or independently of one another, hydrogen or a C₁-C₄ alkyl or hydroxyalkyl radical.

7. Process according to either of Claims 5 and 6, characterized in that the said (poly)amines are chosen from monoethanolamine, diethanolamine, triethanolamine, isopropanolamine and 1,3-propanediamine, alone or mixed.

8. Process according to any one of the preceding claims, characterized in that the concentration of carboxylic acid(s) and/or of their associated salts within the oxidizing composition is between 0.1N and 2N.

9. Process according to Claim 8, characterized in that the said concentration is between 0.2N and 1N.

10. Process according to any one of the preceding claims, characterized in that the pH of the oxidizing composition is between 2.8 and 6.

11. Process according to Claim 10, characterized in that the said pH is between 4 and 5.5.

12. Process according to any one of the preceding claims, characterized in that the hydrogen peroxide concentration in the oxidizing composition is between 1 and 20 volumes.

13. Process according to Claim 12, characterized in that the said concentration is between 1 and 10 volumes.

14. Process according to any one of the preceding claims, characterized in that the oxidizing composition contains adjuvants designed to make it cosmetically acceptable.

15. Process according to Claim 14, characterized in that the said adjuvants are chosen from nonionic, anionic, cationic or amphoteric type surfactants, treatment agents, active ingredients, agents for combating hair loss, anti-dandruff agents, thickeners, suspending agents, sequestering agents, opacifying agents, colorants, sunscreen agents, perfumes and preservatives, alone or mixed.

16. Process according to any one of the preceding claims, characterized in that the oxidizing composition takes the form of a lotion, thickened or otherwise, a milk, a cream or a gel.

17. Process according to any one of the preceding claims, characterized in that the said reducing composition is a carbonate-containing reducing composition.

18. Process according to any one of the preceding claims, characterized in that it comprises the following steps:
(i) the reducing composition is applied to the keratinous material to be treated, the means needed for placing the keratinous material under mechanical tension being deployed before, during or after the said application,
(ii) the keratinous material thus treated is then rinsed,
(iii) the oxidizing composition is applied to the keratinous material thus rinsed,
(iv) the keratinous material thus treated is then separated from the means of placing under tension used in step (i),
(v) and lastly the keratinous material is rinsed again.

19. Process according to any one of the preceding claims, characterized in that the keratinous material to be treated consists of hair.

20. Multi-compartment device or kit, characterized in that it comprises a reducing composition in a first compartment, and an oxidizing composition as defined in any one of Claims 1 to 16 in a second compartment.

21. Device according to Claim 20, characterized in that the said reducing composition is a carbonate-containing reducing composition capable of and designed for carrying out the first step of a permanent-waving/straightening operation.

22. Treatment process for the permanent-reshaping and/or -shape-stabilizing of keratinous materials, of the type consisting, in a first step, in reducing the disulphide bonds of keratin by application of a reducing composition, and then, in a second step, in re-forming the said bonds by application of a hydrogen peroxide-based oxidizing composition, characterized in that a kit as defined in claim 20 or 21 is used.

## Patentansprüche

1. Behandlungsverfahren zur dauerhaften Verformung und/oder Formung von Keratinmaterialien, das darin besteht, in einem ersten Schritt die Disulfidbindungen des Keratins durch Auftragen einer reduzierenden Zusammensetzung zu reduzieren und anschließend in einem zweiten Schritt die Bindungen durch Auftragen einer oxidierenden Zusammensetzung auf der Basis von Wasserstoffperoxid wieder herzustellen,
dadurch gekennzeichnet, daß
die oxidierende Zusammensetzung (i) mindestens eine Carbonsäure und/oder eines der zugehörigen Salze und (ii) mindestens eine alkalisch machende Aminverbindung enthält.

2. Verfahren nach Anspruch 1, dadurch gekennzeichnet, daß die Carbonsäure unter einfachen Carbonsäuren, Polycarbonsäuren, (Poly)hydroxy(poly)carbonsäuren und deren Gemischen ausgewählt ist.

3. Verfahren nach Anspruch 2, dadurch gekennzeichnet, daß die Carbonsäure unter Milchsäure, Weinsäure, Essigsäure, Glykolsäure, Citronensäure und deren Gemischen ausgewählt ist.

4. Verfahren nach Anspruch 3, dadurch gekennzeichnet, daß die Carbonsäure die Citronensäure ist.

5. Verfahren nach einem der vorhergehenden Ansprüche, dadurch gekennzeichnet, daß die alkalisch machende Aminverbindung unter Ammoniak, primären, sekundären oder tertiären (Poly)aminen und deren Gemischen ausgewählt ist.

6. Verfahren nach Anspruch 5, dadurch gekennzeichnet, daß die Polyamine der folgenden allgemeinen Formel entsprechen: 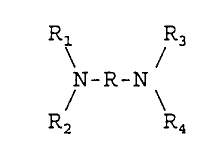 worin R eine gegebenenfalls mit einer Hydroxygruppe oder C₁₋₄-Alkylgruppe substituierte Propylengruppe ist und R₁, R₂, R₃ und R₄ gleichzeitig oder unabhängig voneinander Wasserstoff, C₁₋₄ Alkyl oder C₁₋₄ Hydroxyalkyl bedeuten.

7. Verfahren nach einem der Ansprüche 5 oder 6, dadurch gekennzeichnet, daß die (Poly)amine unter Monoethanolamin, Diethanolamin, Triethanolamin, Isopropanolamin und 1,3-Diaminopropan ausgewählt werden, die allein oder im Gemisch verwendet werden.

8. Verfahren nach einem der vorhergehenden Ansprüche, dadurch gekennzeichnet, daß die Konzentration der Carbonsäure oder Carbonsäuren und/oder der Salze dieser Carbonsäuren in der oxidierenden Zusammensetzung im Bereich von 0,1 bis 2 N liegt.

9. Verfahren nach Anspruch 8, dadurch gekennzeichnet, daß die Konzentration im Bereich von 0,2 bis 1 N liegt.

10. Verfahren nach einem der vorhergehenden Ansprüche, dadurch gekennzeichnet, daß der pH-Wert der oxidierenden Zusammensetzung im Bereich von 2,8 bis 6 liegt.

11. Verfahren nach Anspruch 10, dadurch gekennzeichnet, daß der pH-Wert im Bereich von 4 bis 5,5 liegt.

12. Verfahren nach einem der vorhergehenden Ansprüche, dadurch gekennzeichnet, daß die Konzentration des Wasserstoffperoxids in der oxidierenden Zusammensetzung im Bereich von 1 bis 20 Volumina liegt.

13. Verfahren nach Anspruch 12, dadurch gekennzeichnet, daß die Konzentration im Bereich von 1 bis 10 Volumina liegt.

14. Verfahren nach einem der vorhergehenden Ansprüche, dadurch gekennzeichnet, daß die oxidierende Zusammensetzung Zusätze enthält, die dazu dienen, sie kosmetisch akzeptabel zu machen.

15. Verfahren nach Anspruch 14, dadurch gekennzeichnet, daß die Zusätze unter den nichtionischen, anionischen, kationischen oder amphoteren grenzflächenaktiven Mitteln, Behandlungsmitteln, Wirkstoffen, Mitteln gegen Haarausfall, Mitteln gegen Schuppen, Verdickungsmitteln, Suspendiermitteln, Maskierungsmitteln, Trübungsmitteln, Färbemitteln, Sonnenschutzfiltern, Parfums, Konservierungsmitteln und deren Gemischen ausgewählt sind.

16. Verfahren nach einem der vorhergehenden Ansprüche, dadurch gekennzeichnet, daß die oxidierende Zusammensetzung in Form einer gegebenenfalls verdickten Lotion, einer Milch, einer Creme oder eines Gels vorliegt.

17. Verfahren nach einem der vorhergehenden Ansprüche, dadurch gekennzeichnet, daß die reduzierende Zusammensetzung eine carbonathaltige reduzierende Zusammensetzung ist.

18. Verfahren nach einem der vorhergehenden Ansprüche, dadurch gekennzeichnet, daß es die folgenden Schritte umfaßt:
(i) Auftragen der reduzierenden Zusammensetzung auf das zu behandelnde Keratinmaterial, wobei die Mittel, die erforderlich sind, um das Keratinmaterial unter mechanische Spannung zu setzen, vor, während oder nach dem Auftragen angebracht werden,
(ii) Spülen des so behandelten Keratinmaterials,
(iii) Auftragen der oxidierenden Zusammensetzung auf das so gespülte Keratinmaterial,
(iv) Trennen des so behandelten Keratinmaterials von den in Schritt (i) verwendeten Mitteln, mit denen es unter Spannung gesetzt wird,
(v) schließlich nochmaliges Spülen des Keratinmaterials.

19. Verfahren nach einem der vorhergehenden Ansprüche, dadurch gekennzeichnet, daß das zu behandelnde Keratinmaterial aus Haaren besteht.

20. Vorrichtung mit mehreren Abteilungen oder "Kit", dadurch gekennzeichnet, daß sie in einer ersten Abteilung eine reduzierende Zusammensetzung und in einer zweiten Abteilung eine oxidierende Zusammensetzung nach einem der Ansprüche 1 bis 16 enthält.

21. Vorrichtung nach Anspruch 20, dadurch gekennzeichnet, daß die reduzierende Zusammensetzung eine carbonathaltige Zusammensetzung ist, die zur Verwendung im ersten Schritt einer Dauerwellverformung verwendet werden soll und dafür geeignet ist.

22. Behandlungsverfahren zur dauerhaften Verformung und/oder Formung von Keratinmaterialien, das darin besteht, in einem ersten Schritt die Disulfidbindungen des Keratins durch Auftragen einer reduzierenden Zusammensetzung zu reduzieren und anschließend in einem zweiten Schritt die Bindungen durch Auftragen einer oxidierenden Zusammensetzung auf der Basis von Wasserstoffperoxid wieder herzustellen, dadurch gekennzeichnet, daß ein "Kit" nach einem der Ansprüche 20 oder 21 verwendet wird.
